(19) Europäisches Patentamt / European Patent Office / Office européen des brevets

(11) **EP 3 083 971 B1**

(12) **EUROPEAN PATENT SPECIFICATION**

(45) Date of publication and mention of the grant of the patent:
**02.05.2018 Bulletin 2018/18**

(21) Application number: **14836961.4**

(22) Date of filing: **22.12.2014**

(51) Int Cl.:
*C12P 7/08* (2006.01)    *C12P 5/02* (2006.01)
*C12P 3/00* (2006.01)    *C02F 11/04* (2006.01)
*B01J 27/24* (2006.01)    *B01J 31/06* (2006.01)

(86) International application number:
**PCT/EP2014/079015**

(87) International publication number:
**WO 2015/092067 (25.06.2015 Gazette 2015/25)**

(54) **PROCESS FOR THE PRODUCTION OF BIOFUEL BY ANAEROBIC DIGESTION USING A HYDROLYTIC ENZYME AND A POLYAMINE**

VERFAHREN ZUR HERSTELLUNG VON BIOKRAFTSTOFF DURCH ANAEROBISCHE GÄRUNG UNTER VERWENDUNG EINES HYDROLYTISCHEN ENZYMS UND EINES POLYAMINS.

PROCÉDÉ POUR PRODUCTION D'UN BIOCARBURANT PAR DIGESTION ANAÉROBIE EN UTILISANT D'UNE ENZYME HYDROLYTIQUE ET D'UNE POLYAMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2013 EP 13198792**

(43) Date of publication of application:
**26.10.2016 Bulletin 2016/43**

(73) Proprietor: **Methodo Chemicals S.r.l.**
**42017 Novellara (IT)**

(72) Inventor: **BELTRAMI, Andrea**
**I-42017 Novellara (IT)**

(74) Representative: **Palladino, Saverio Massimo et al**
**Notarbartolo & Gervasi S.p.A.**
**Corso di Porta Vittoria, 9**
**20122 Milano (IT)**

(56) References cited:
- **KHEMKHAO ET AL: "Effect of chitosan on UASB treating POME during a transition from mesophilic to thermophilic conditions", BIORESOURCE TECHNOLOGY, vol. 102, 2011, pages 4674-4681, XP028154110,**
- **BASILE ET AL: "The effect of the surface charge of hydrogel supports on thermophilic biohydrogen production", BIORESOURCE TECHNOLOGY, vol. 101, 2010, pages 4386-4394, XP026953074,**
- **LAKANIEMI ET AL: "Biogenic hydrogen and methane production from Chlorella vulgaris and Dunaliella tertiolecta biomass", BIOTECHNOLOGY FOR BIOFUELS, vol. 4, 2011, pages 34(1)-45(12), XP021111198,**
- **CARVER ET AL: "Thermophilic, anaerobic co-digestion of microalgal biomass and cellulose for H2 production", BIODEGRADATION, vol. 22, 2011, pages 805-814, XP019910444,**
- **NO ET AL: "Application of chitosan for treatment of wastewaters", REVIEWS OF ENVIRONMENTAL CONTAMINATION AND TOXICOLOGY, vol. 163, 2000, pages 1-28, XP001525071,**
- **DARIAS ET AL: "Functional stabilization of cellulase by covalent modification with chitosan", JOURNAL OF CHEMICAL TECHNOLOGY AND BIOTECHNOLOGY, vol. 76, 2001, pages 489-493, XP001091997,**
- **MANRICH ET AL: "Immobilization and stabilization of xylanase by multipoint covalent attachment on agarose and on chitosan supports", APPLIED BIOCHEMISTRY AND BIOTECHNOLOGY, vol. 161, 2010, pages 455-467, XP002738172,**

**(Cont. next page)**

- NWAGU ET AL: "Improved yield and stability of amylase by multipoint covalent binding on polyglutaraldehyde activated chitosan beads: Activation of denatured enzyme molecules by calcium ions", PROCESS BIOCHEMISTRY, vol. 48, 25 May 2013 (2013-05-25), pages 1031-1038, XP028673150,

**Description**

**Field of the invention**

The present invention relates to the use of a polyamine in processes for producing biofuels by anaerobic digestion of organic biomasses. In particular, the presence of a polyamine allows to increase the action of hydrolytic enzymes, while facilitating and promoting the process of hydrolysis and saccharification, but especially increasing the final yields in biofuels by reduction of the output non-digested biomass.

**State of the art**

Recently, the agricultural and zootechnical production line has been directly affected by energy productions: renewable energies and green economy have driven the agricultural world to producing electrical energy or biofuels from renewable sources through different forms of incentive.

Renewable energy plants of the agricultural type, excluding photovoltaic ones, are in particular directed to producing methane and to producing combustible ethanol. Both these processes have as a common factor the anaerobic digestion of organic biomasses of various kinds: food waste, organic waste, zootechnical slurry, but also agricultural raw materials (agricultural biomasses) and animal feed.

The anaerobic digestion consists in a complex and non-standardized (from neither the chemical-physical nor the microbiological point of view) microbiological process which envisages the conversion of complex organic polymers (sugars, starches, fibres) into organic acids, hydrogen and carbon dioxide which, depending on the microbiological strain, are in turn converted into the so called biofuels (typically methane and ethanol); the cause of the lack of standardization is the variety of plant types and of selection of plant conduction which, therefore, affects the development of variable microbiological floras.

One of the main problems in this type of plants is the low degradability of the components more resistant to fermentations, particularly fibrous and structural cellulose-based components. The cause of the resistance may be a poor microbiological efficiency, the size of the plant (and therefore of its hydraulic flows) or the type of matrix (higher or lower presence of lignin, for example).

The object of the present invention is therefore to improve the efficiency of such processes, while increasing at the same time the yields of the final biofuel. Khemkhao et al, Bioresource Technology 102 (2011) 4674-4681, discloses the effect of chitosan on USAB (upflow anaerobic sludge bed) treating POME (palm oil mill effluent) with an increased biogas production.

**Summary of the invention**

The object indicated above has been achieved by a process for producing biofuel by anaerobic digestion of biomass in the presence of at least one hydrolytic enzyme and at least one polyamine, as per claim 1. In a second embodiment, the use of a composition consisting of at least one hydrolytic enzyme and at least one polyamine is provided, as per claim 13. For the purposes of the present invention, the term "biofuel" means any combustible gas or liquid derived from degradation of biological starting materials, such as, but not limited to, biogas, biomethane, biomethanol, bioethanol, biobutanol, bioacetone, bio-dimethyl ether, hydrogen, or mixtures thereof or liquid or gaseous mixtures containing at least one of these compounds. The term "biomass" means cellulose or pastes thereof, sludges, or treatment water, molasses, starches or flours from crops, silage crops, crops in general (also not in silage) or in the form of animal feed or pellets/flakes, slurry, exhausted zootechnic litters and manure containing crude cellulose, sewage and waste waters, non-treated or pre-treated sludge, sludge and/or organic fractions of urban solid waste and/or waste products or by-products of the food industry or transformation of agricultural products or animals, and mixtures thereof.

The term "hydrolytic enzyme" means xylanase, amylase, glucanase, cellulase, or a mixture thereof, isolated and purified or supported on plant material or inert mineral, coming from industrial biotechnological GMO and non-GMO productions, or from specific microorganisms cultured on dedicated liquid or solid substrates, such as bacteria, fungi, or yeasts.

"Polyamine" means a polymer having 2 to 1,000 amino groups, preferably 2 to 100 amino groups. Examples of polyamines include chitosan, ethylenediamine, diethylenetriamine, triethylenetetramine, tetraethylenepentamine, pentaethylenehexamine, dipropylenetriamine, tripropylenetetramine, tetrapropylenepentamine, pentapropylenehexamine, polyethylenimine having an average degree of polymerization (corresponding to the number of nitrogen atoms) of, for example, 10, 35, 50 or 100, and also polyamines obtained by reacting oligoamines (with chain extension) with acrylonitrile and subsequent hydrogenation, for example N,N'-bis-(3-aminopropyl)ethylenediamine.

**Detailed description of the invention**

The invention therefore concerns a process for producing biofuel by anaerobic digestion of biomass wherein said biofuel is a combustible gas or liquid derived from degradation of biological starting materials, said process comprising the steps of:

> i) feeding biomass to an anaerobic digester,
> ii) feeding digestate comprising at least one hydrolytic enzyme,
> iii) adding at least one polyamine to increase the hydrolysis of the complex polymers present in the biomass
> iv) allowing the resulting mass to react in anaerobic conditions, and
> v) separating the biofuel obtained, wherein steps i), ii) and iii) are carried out, in any order, before steps iv) and v).

In fact, it has been surprisingly observed that the presence of at least one polyamine allows to advantageously increase the efficiency and yield of final biofuel.

Digestate is the fermenting medium of the process of the invention and is obtained as a process by-product of anaerobic digestion. Digestate is a material that, as compared to the starting biomasses, is homogeneous, with a higher humidity degree because part of the dry matter has been degraded biologically, i.e. degraded by bacteria. The remaining organic matter is more stable and contains elements of fertility, such as nitrogen, phosphorus and potassium.

The efficiency of anaerobic digestion of the process of the invention is determined by the effective yield in biofuel as compared to the theoretical yield deriving from the starting biomass.

The combined use of at least one hydrolytic enzyme and at least one polyamine surprisingly increases the hydrolysis of the complex polymers present in the biomass, such as starches, cellulose, glucanes, xylanes hemicelluloses, proteins, into simpler compounds, such as sugars, organic acids, amino acids.

For industrial plants having structural, conduction or management problems, the yields may be disadvantageously lower than expected; this happens especially when the biomass turnover within the process of anaerobic digestion is very rapid.

On the contrary, the process of the invention also advantageously addresses this problem by offering higher yields owing to the presence of at least one polyamine. This adds to the further advantage that the addition of said at least one polyamine does not require dedicated facilities or equipment, nor particular modifications to pre-existing biofuel production plants.

Furthermore, the use of said at least one polyamine does not cause the formation of substances considered detrimental to the environment and, as being biodegradable, the waste products coming from the anaerobic digester, in turn, are not dangerous for the health or the environment.

The process of the invention may be either a continuous or a batch process, both with complete mix digesters and plug-flow digesters. The temperatures are variable from RT (= no heating) up to 60 °C (in the art, the plants are said to be operating in psychrophyly, mesophyly and thermophyly). The dry matter may be from about 1 % to 50% by weight on the weight of the biomass.

Preferably, steps i)-iii) are carried out simultaneously or substantially simultaneously.

Preferably, said at least one polyamine is at a concentration of 50-1000 ppm, more preferably 50-500 ppm, and even more preferably 50-250 ppm, in the anaerobic digester.

Such polyamine concentration values can be calculated and verified by known methods, such as the measurement of viscosity, as per Huggins and Kraemer equations (Rosen S.L. Fundamental principles of polymeric materials 2nd Ed.: John Wiley and Sons: New York. 1993: 58-68).

As usual in the field, to better characterize the polyamine the measure is repeated at different polyamine concentration values.

In preferred embodiments, said at least one polyamine has a molecular weight of 1,000-80,000 Da, a viscosity (Brookfield) of 150-200 cps (150-200 mPas) (measured at 5 g/l), 70-100 cps (70-100 mPas) (2.5 g/l), and 25-50 (25-50 mPas) (1 g/l), and a bulk density of 0.50-0.95 g/cm$^3$.

In a particularly preferred embodiment, said at least one polyamine has a molecular weight of 1,000-80,000 Da, a viscosity (Brookfield) of 180 cps (180 mPas) (measured at 5 g/l), 85 cps (85 mPas) (2.5 g/l), and 35 cps (35 mPas) (1 g/l), and a bulk density of 0.85 g/cm$^3$.

Optionally, a further amount of one or more hydrolytic enzymes may be added to the anaerobic digester, if the digestate itself contains them in an insufficient amount, or in order to increase the effectiveness of the process.

Generally, the greater the amount of enzyme, the greater is the fermentation activity and thus the final production of biogas. Therefore, in order to increase the performance of the process, it may be envisaged the feeding into the digester of an additional amount of at least 10 g of enzyme(s) per ton of biomass. The dosing of this additional amount of enzyme(s) may take place during step iv) of the process, or as part of step iii) of the process, as specified below.

In a preferred embodiment, said at least one polyamine and said additional amount of one or more hydrolytic enzymes are in the form of an adduct or are chemically bound to one another, therefore in step iii) also the addition of a further amount of one or more hydrolytic enzymes is simultaneously carried out.

In another embodiment, in step iii) also cationic or anionic polyacrylamide, carboxymethylcellulose, or a mixture thereof, is further added. Preferably, said polyacrylamide has a molecular weight of 6-10 MDa.

More preferably, said cationic or anionic polyacrylamide, carboxymethylcellulose, or a mixture thereof, is at a concentration of 50-1000 ppm in the anaerobic digester. The measurement of such concentration may be assessed as reported above for the at least one polyamine.

Similarly, also said cationic or anionic polyacrylamide, carboxymethylcellulose, or a mixture thereof, and said at least one hydrolytic enzyme may be in the form of an adduct or chemically bound to one another.

According to another aspect, the present invention relates to the use of a composition consisting of at least one hydrolytic enzyme and at least one polyamine, with the optional addition of a cationic or anionic polyacrylamide, carboxymethylcellulose, or a mixture thereof, to increase the hydrolysis of complex polymers contained in the biomass in the process according to the process claims. In a preferred embodiment, said at least one hydrolytic enzyme and said at least one polyamine, and possibly said cationic or anionic polyacrylamide, carboxymethylcellulose or a mixture thereof, are in the form of an adduct or are chemically bound to one another.

Preferably, said composition is in solid form, more preferably in the form of powder, beads, flakes, capsules, sticks, bars, pellets, multiparticulate matter, micronized particulate matter, agglomerates, micro-granules, granules, or tablets. Alternatively, said composition is in the form of a liquid, gel, suspension or emulsion.

Optionally, said composition may be coated by means of a coating or microencapsulated in order to obtain an extended release formulation.

In order to test the effectiveness of the process of the invention, a comparison test was carried out between a process of methanation in the absence of polyamine (control - C) and a process of methanation in the presence of a polyamine, in particular at a concentration of about 100 ppm (test - T), according to the invention.

The reference protocol selected was the UNI EN ISO 11734:2004 standard adapted to carry out the test with the addition of an agricultural raw material to the fermentation medium, that is, corn silage.

A mixture of organic biomass and fresh digestate, proportioned according to the requirement of the standard at section 6.3, in order to reach 100 mg/L of organic carbon (OC), is incubated at 38 $\pm$ 2 °C in hermetically closed containers for a period of about 60 days.

In the test container, the increase in the head space pressure, due to the generation of carbon dioxide ($CO_2$) and methane ($CH_4$) was determined quantitatively. The pattern of biodegradation may be followed by carrying out measurements at intermediate times of the sole gas production. In order to have additional information, it is possible to determine primary biodegradation at the beginning and at the end of the test, by means of specific analyses on the starting and final fermentation medium.

The thus adapted test reflects fermentation systems entirely similar to industrial ones for the production of biofuels.

The stock solution is the basic medium to which the other components are to be added. In particular, as indicated in the standard above, two stock solutions were prepared:

- Control Solution, as a standard dilution solution such that, at preset amounts, it contains the following constituents:

  Anhydrous potassium dihydrogen phosphate ($KH_2PO_4$) 0.27 g Dodecahydrate disodium phosphate ($Na_2HPO_4\cdot12H_2O$) 1.12 g Ammonium chloride ($NH_4Cl$) 0.53 g Dihydrate calcium chloride ($CaCl_2\cdot2H_2O$) 0.075 g Hexahydrate magnesium chloride ($MgCl_2\cdot6H_2O$) 0.10 g Tetrahydrate iron chloride (II) ($FeCl_2\cdot4H_2O$) 0.02 g Resazurin (oxygen indicator) 0.001 g Nonahydrate sodium sulfide ($Na_2S\cdot9H_2O$) 0.1 g

- TEST Solution, envisages the same composition of the Control Solution with the addition of polyamines in order to obtain an emulsion. The amount of polyamines added to the fermentation medium was such that at the final volume, the polyamines were at a concentration of about 100 ppm. The polyamine used for preparing the TEST Solution object of this example is a linear polyamine obtained by polycondensation of dimethyl amine and epichlorohydrin.

Both Control and Test solutions are prepared with de-oxygenated water (containing less than 2 mg/l of dissolved organic carbon (DOC)) quantum satis to reach a volume of 1 L. In order to achieve anoxic conditions, in the medium, immediately before its use, it is bubbled nitrogen for about 20 min, so as to eliminate oxygen. The pH of the medium is adjusted with diluted mineral acid or base, if necessary, up to 7 $\pm$ 0.2.

Polyamines

The polyamines used have a molecular weight of 1000-8000 Da, a viscosity (Brookfield) of 180 cps (180 mPas) when measured at 5 g/l, 85 cps (85 mPas) (measured at 2.5 g/l), and 35 cps (35 mPas) (measured at 1 g/l), and a bulk density of 0.85 g/cm$^3$.

Digestate

A sufficient amount of digestate is provided coming from a plant powered in a way that is as similar as possible to the organic biomass fed to the test (example: a plant powered at 70% of corn silage and 30% of slurry if a biomass mixture of fresh zootechnical effluents and corn silage is to be tested).

Biomass

The biomass may be in the form of a stock solution, a suspension, an emulsion, or directly as a solid or liquid, so that it produces a test concentration of 100 mg/l of organic carbon. For test compounds that are not sufficiently water-soluble, reference can be made to the ISO 10634 standard, but without using organic compounds such as chloroform or carbon tetrachloride which, as is known, inhibit the production of methane.

Inoculum

Right before use, wash the digestate so as to reduce the concentration of Inorganic Carbon (IC) in the test solution at less than 10 mg/l, initially spinning in test tubes, hermetically closed, at a relatively low speed (for example at 3,000 rpm), for a maximum time of 5 min. Suspend in the dilution medium, oxygen-free, the bead precipitate, spin and eliminate washes. If the IC value has not been lowered sufficiently, wash the sludge two more times at most. Lastly, suspend the precipitate in the required volume of the dilution medium and determine the concentration of the total solid content. The final concentration of the total solid content should be in range of 1 g/l to 3 g/l. Perform these operations so that the contacting of the sludge with oxygen is reduced to the minimum (for example operating under nitrogen atmosphere). A Control inoculum, produced with the Control Solution, and a Test inoculum, produced with the TEST Solution, will be thus obtained.

Incubation or water-bath equipment, or sand, are thermally controlled at 35 ± 2 °C. A needle pressure measurement device is also used, for example a manometer connected to a suitable syringe needle; a gas-tight three-way valve facilitate the release of excess pressure. The device must be used and calibrated based on the manufacturer's instructions. It is necessary to maintain, to the lowest value possible, the inner volume of the valve and the piping of the pressure transducer, so that any errors introduced by disregarding the volume of the equipment are negligible.

A carbon analyzer is further used, adapted to directly determine organic carbon in the range of 1 mg/l to 200 mg/l.

**Method of comparison**

Carry out the following starting procedures, using techniques suitable for maintaining the contact between digested sludge and oxygen to the lowest values possible, for example operating in an anaerobic chamber under nitrogen atmosphere, or purging the bottles with nitrogen.

*Preparation of test samples and control samples*

Mix, in appropriate test containers the inoculum, about 100 g of test biomass and digestate. The final test concentration of carbon usually must be of 100 mg/l, therefore, the amount of biomass will be weighted based on this parameter. At the end of the preparation, two final mixtures will be obtained, one produced with the base inoculum (Control) and one produced with an inoculum including polyamines at the concentration of about 100 ppm (TEST).

Make sure that the total liquid volume (or initial Volume, Vi) and the volume of head space (Vh) are the same in all containers. Note down Vi and Vh. If necessary, add anoxic test medium. Hermetically close each of the containers with the gas-impermeable septum and incubate at 38 °C. A regular mixing of incubated bottles is advisable. Observe the containers after an incubation time of 24 h to 48 h. Discard the containers in which the content has a clear pink color of the supernatant, that is if resazurin has shown color variation, indicating the presence of oxygen. While small amounts of oxygen may be tolerated by the system, higher concentrations may significantly inhibit the development of the anaerobic biodegradation. Mix the content of each container by stirring, or shaking for a few minutes at least one or two times per week, and before each pressure measurement. Measure gas pressure, for example passing the syringe needle, connected to the pressure monitoring device, through the partition. Record pressure in millibar. Stirring brings the inoculum back to the suspension and ensures gas balance. While measuring the pressure, the gas in the head space must be kept to the same temperature as that of digestion. Attention should be paid to prevent water from entering the syringe needle. In that case, dry any wet parts and insert a new needle. In order to obtain the value of gas pressure, measure the pressure within the containers once a week, release the excess gas in the atmosphere or only measure the pressure at the end of the test, in order to determine the amount of biogas produced.

Complete the test after a period of incubation of 60 days, unless the biodegradation curve obtained from the pressure measurement has not reached the plateau stage, that is, the stage wherein the maximum degradation has been reached, indicating a sufficient degree of biodegradation (>50%) to complete the test. If, at the end of the usual period of incubation, the plateau stage has not been clearly reached, the test should be continued until reaching such stage.

### Analysis of results

Take a sample of the final mixture at the beginning of the test and at the end of the test (once digestion has completed).

Measure total solids (SS%) by stove drying, and volatile solids (SV%) by incineration in muffle furnace.

BIOGAS PRODUCTIONS measured by normal cubic metres of biogas per ton of volatile solids dissolved into the fermentation medium:

CONTROL = 592.2 Nm$^3$/tsv
TEST = 629.1 Nm$^3$/tsv

DEGREE OF BIODEGRADATION: MAXIMUM ANAEROBIC DEGRADABILITY measured as a percentage of volatile solids converted into the fermentation medium:

CONTROL = 75.7%
TEST = 81.4%

50% DEGRADABILITY TIMES, measured as the time needed to degrade 50% of volatile solids globally converted into gas:

CONTROL = 6.4 days
TEST = 5.9 days

The measurement of the volumes of biogas produced shows that in the TEST sample an increase of biogas production by about 6%, an increase in overall degradability of organic matter by 5.7% and a reduction in degradability times by about 12 hours are achieved.

In the hypothesis that the ashes are not converted to biogas, from the analysis of the digestate the phenomenon of concentration of solids into the fermentation medium by biogas release is considered and therefore, the following is obtained:

$$Concentration\ factor\ of\ medium = \frac{Ashes\ (OUT)}{Ashes\ (IN)}$$

$$\%\ PROCESS\ EFF. = \left[1 - \left(\frac{\left|\frac{SV(OUT)}{(Conc.\ Fatt.)}\right|}{SV(IN)}\right)\right] * 100$$

CONTROL (C) test process efficiency:

|  | IN |  |
|---|---|---|
| ST [g/kg] | SV [g/kg] | Ashes [g/kg] |
| 49.94 | 37.66 | 12.28 |

(continued)

| OUT | | |
|---|---|---|
| ST [g/kg] | SV [g/kg] | Ashes [g/kg] |
| 37.60 | 25.91 | 11.69 |

Process efficiency (C) = 27.73%

TEST (T) test process efficiency:

| IN | | |
|---|---|---|
| ST [g/kg] | SV [g/kg] | Ashes [g/kg] |
| 63.27 | 49.81 | 13.46 |
| OUT | | |
| ST [g/kg] | SV [g/kg] | Ashes [g/kg] |
| 42.30 | 29.30 | 13.00 |

Process efficiency (T) = 39.10%

The above-reported test shows how the process of the invention in the presence of polyamines at the concentration of about 100 ppm has increased the hydrolytic efficiency by more than 11% with an increase in biogas production by about 6% as compared to the control process. The increase in the process efficiency is considered to be significant and capable of widely accounting for the cost deriving from the use of polyamines.

Based on the results obtained, the possibility of using polyamines in anaerobic fermentations is advantageous especially in those cases in which the output waste products (sludge or digestates) still have a share of "non-digested", typically cellulose-based, organic matter.

**Claims**

1. A process for the production of a biofuel by anaerobic digestion of biomass wherein said biofuel is a combustible gas or liquid derived from degradation of biological starting materials, said process comprising the steps of:

   i) feeding biomass to an anaerobic digester,
   ii) feeding digestate comprising at least one hydrolytic enzyme,
   iii) adding at least one polyamine to increase the hydrolysis of the complex polymers present in the biomass,
   iv) allowing the resulting mass to react in anaerobic conditions, and
   v) separating the biofuel obtained,

   wherein steps i), ii) and iii) are carried out, in any order, before steps iv) and v).

2. The process of claim 1, wherein steps i), ii) and iii) are carried out simultaneously or substantially simultaneously.

3. The process of any one of claims 1 or 2, wherein said at least one polyamine is at a concentration of 50-1000 ppm in the anaerobic digester.

4. The process of any one of claims 1 to 3, wherein said at least one polyamine is at a concentration of 50-500 ppm in the anaerobic digester.

5. The process of claim 4, wherein said at least one polyamine has a molecular weight of 1,000-80,000 Da, a Brookfield viscosity of 150-200 cps (150-200 mPas) measured at 5 g/l, 70-100 cps (70-100 mPas) measured at 2.5 g/l, and 25-50 cps (25-50 mPas) measured at 1 g/l, and a bulk density of 0.50-0.95 g/cm$^3$.

6. The process of any one of claims 1 to 5, wherein a further amount of one or more hydrolytic enzymes is added to the biomass.

7. The process of claim 6, wherein said at least one polyamine and said one or more additional hydrolytic enzymes are in the form of an adduct or are chemically bound to one another.

8. The process of any one of claims 1 to 7, wherein said at least one polyamine is selected from chitosan, ethylenediamine, diethylenetriamine, triethylenetetraamine, tetraethylenepentamine, pentaethylenehexamine, dipropylenetriamine, tripropylenetetramine, tetrapropylenepentamine, pentapropylenehexamine, and polyethyleneimine.

9. The process of any one of claims 1 to 8, wherein said hydrolytic enzyme is xinalase, amylase, glucanase, cellulase or a mixture thereof.

10. The process of any one of claims 1 to 9, wherein in step iii) a cationic or anionic polyacrylamide, carboxymethylcellulose, or a mixture thereof is further added.

11. The process of claim 9, wherein said cationic or anionic polyacrylamide, carboxymethylcellulose or a mixture thereof is at a concentration of 50-1000 ppm in the anaerobic digester.

12. The process of any one of claims 10 or 11, wherein said cationic or anionic polyacrylamide, carboxymethylcellulose or a mixture thereof and said one or more additional hydrolytic enzymes are in the form of an adduct or are chemically bound to one another.

13. Use of a composition consisting of at least one hydrolytic enzyme and at least one polyamine to increase the hydrolysis of complex polymers contained in the biomass in the process according to anyone of claims 1-12.

14. Use of the composition of claim 13, wherein said at least one hydrolytic enzyme and at least one polyamine are in the form of an adduct or are chemically bound to one another.

15. Use of the composition of any one of claims 13 or 14, further comprising a cationic or anionic polyacrylamide, carboxymethylcellulose, or a mixture thereof.

16. Use of the composition of any one of claims 13 to 15, said composition being in the solid form selected from powder, beads, flakes, capsules, sticks, bars, pellets, multiparticulate matter, micronized particulate matter, agglomerates, microgranules, granules or tablets.

17. Use of the composition of any one of claims 13 to 15, said composition being in the form of a liquid, gel, suspension or emulsion.

**Patentansprüche**

1. Verfahren zur Herstellung eines Biokraftstoffs durch anaeroben Abbau von Biomasse, wobei der Biokraftstoff brennbares Gas oder Flüssigkeit ist, das aus dem Abbau von biologischem Ausgangsmaterial stammt, wobei das Verfahren folgende Schritte aufweist:

i) Zuführen von Biomasse an einen anaeroben Fermenter,
ii) Zuführen von Gärgut, das zumindest ein hydrolytisches Enzym aufweist,
iii) Zugabe von zumindest einem Polyamin, um die Hydrolyse der in der Biomasse enthaltenen komplexen Polymere zu verbessern,
iv) reagieren lassen der resultierenden Masse in anaeroben Bedingungen und
v) Abscheiden des erhaltenen Biokraftstoffs,

wobei die Schritte i), ii) und iii) in jeglicher Reihenfolge, aber vor den Schritten iv) und v) durchgeführt werden.

2. Verfahren gemäß Anspruch 1, wobei die Schritte i), ii) und iii) gleichzeitig oder im Wesentlichen gleichzeitig durchgeführt werden.

**3.** Verfahren gemäß einem der Ansprüche 1 oder 2, wobei das zumindest eine Polyamin in einer Konzentration von 50 bis 1.000 ppm in dem anaeroben Fermenter vorliegt.

**4.** Verfahren gemäß einem der Ansprüche 1 bis 3, wobei das zumindest eine Polyamin in einer Konzentration von 50 bis 500 ppm in dem anaeroben Fermenter vorliegt.

**5.** Verfahren gemäß Anspruch 4, wobei das zumindest eine Polyamin aufweist:

- ein Molekulargewicht von 1.000 bis 80.000 Da,
- eine Brookfield-Viskosität von
150 - 200 cps (150 - 200 mPas), gemessen bei 5 g/l,
70 - 100 cps (70 - 100 mPas), gemessen bei 2,5 g/l und
25 - 50 cps (25 - 50 mPas), gemessen bei 1 g/l, sowie
- eine Raumdichte von 0,50 - 0,95 g/cm$^3$.

**6.** Verfahren gemäß einem der Ansprüche 1 bis 5, wobei der Biomasse eine weitere Menge eines oder mehrerer hydrolytischer Enzyme zugegeben wird.

**7.** Verfahren gemäß Anspruch 6, wobei das zumindest eine Polyamin und das eine oder die mehreren zusätzlichen hydrolytischen Enzyme in Form eines Addukts vorliegen oder chemisch miteinander verbunden sind.

**8.** Verfahren gemäß einem der Ansprüche 1 bis 7, wobei das zumindest eine Polyamin ausgewählt wird aus Chitosan, Ethylendiamin, Diethylentriamin, Triethylentetraamin, Tetraethylenpentamin, Pentaethylenhexamin, Dipropylentriamin, Tripropylentetraamin, Tetrapropylenpentamin, Pentapropylenhexamin und Polyethylenimin.

**9.** Verfahren gemäß einem der Ansprüche 1 bis 8, wobei das hydrolytische Enzym Xinalase, Amylase, Glucanase, Cellulase oder eine Mischung daraus ist.

**10.** Verfahren gemäß einem der Ansprüche 1 bis 9, wobei in Schritt iii) des Weiteren kationisches oder anionisches Polyacrylamid, Carboxymethylcellulose oder eine Mischung daraus zugegeben wird.

**11.** Verfahren gemäß Anspruch 9, wobei das kationische oder anionische Polyacrylamid, Carboxymethylcellulose oder eine Mischung daraus in einer Konzentration von 50 bis 1.000 ppm in dem anaeroben Fermenter vorliegt.

**12.** Verfahren gemäß einem der Ansprüche 10 oder 11, wobei das kationische oder anionische Polyacrylamid, Carboxymethylcellulose oder eine Mischung daraus und das eine oder die mehreren zusätzlichen hydrolytischen Enzyme in Form eines Addukts vorliegen oder chemisch miteinander verbunden sind.

**13.** Verwendung einer Zusammensetzung, bestehend aus zumindest einem hydrolytischen Enzym und zumindest einem Polyamin zur Verbesserung der Hydrolyse komplexer Polymere, die in der Biomasse des Verfahrens gemäß der Ansprüche 1 bis 12 enthalten sind.

**14.** Verwendung der Zusammensetzung gemäß Anspruch 13, wobei das zumindest eine hydrolytische Enzym und das zumindest eine Polyamin in Form eines Addukts vorliegen oder chemisch miteinander verbunden sind.

**15.** Verwendung der Zusammensetzung gemäß einem der Ansprüche 13 oder 14, des Weiteren aufweisend ein kationisches oder anionisches Polyacrylamid, Carboxymethylcellulose oder eine Mischung daraus.

**16.** Verwendung der Zusammensetzung gemäß einem der Ansprüche 13 bis 15, wobei die Zusammensetzung in fester Form ausgewählt wird aus Pulver, Perlen, Flocken, Kapseln, Stielen, Stäben, Pellets, multipartikulärem Material, mikronisierter Partikelmasse, Agglomeraten, Mikrogranulat, Granulaten oder Tabletten.

**17.** Verwendung der Zusammensetzung gemäß einem der Ansprüche 13 bis 15, wobei die Zusammensetzung in Form einer Flüssigkeit, eines Gels, einer Suspension oder einer Emulsion vorliegt.

**Revendications**

1. Procédé de production d'un biocarburant par digestion anaérobie de biomasse, dans lequel ledit biocarburant est un gaz ou liquide combustible dérivé de la dégradation des matières premières biologiques, ledit procédé comprenant les étapes suivantes :

   i) fournir la biomasse à un digesteur anaérobie,
   ii) fournir le digestat comprenant au moins une enzyme hydrolytique,
   iii) ajouter au moins une polyamine pour accroître l'hydrolyse des polymères complexes présents dans la biomasse,
   iv) permettre à la masse résultante de réagir dans des conditions anaérobies, et
   v) séparer le biocarburant obtenu,

   dans lequel les étapes i), ii) et iii) sont réalisées, dans tout ordre, avant les étapes iv) et v).

2. Procédé selon la revendication 1, dans lequel les étapes i), ii) et iii) sont réalisées simultanément ou sensiblement simultanément.

3. Procédé selon l'une quelconque des revendications 1 ou 2, dans lequel ladite au moins une polyamine se trouve en une concentration de 50 à 1000 ppm dans le digesteur anaérobie.

4. Procédé selon l'une quelconque des revendications 1 à 3, dans lequel ladite au moins une polyamine se trouve en une concentration de 50 à 500 ppm dans le digesteur anaérobie.

5. Procédé selon la revendication 4, dans lequel ladite au moins une polyamine a une masse moléculaire de 1000 à 80 000 Da, une viscosité Brookfield de 150 à 200 cps (150 à 200 mPas) mesurée à 5 g/l, de 70 à 100 cps (70 à 100 mPas) mesurée à 2,5 g/l, et de 25 à 50 cps (25 à 50 mPas) mesurée à 1 g/l, et une densité apparente de 0,50 à 0,95 g/cm$^3$.

6. Procédé selon l'une quelconque des revendications 1 à 5, dans lequel une quantité supplémentaire d'une ou plusieurs enzymes hydrolytiques est ajoutée à la biomasse.

7. Procédé selon la revendication 6, dans lequel ladite au moins une polyamine et lesdites une ou plusieurs enzymes hydrolytiques supplémentaires se trouvent sous la forme d'un produit d'addition ou sont chimiquement liées les unes aux autres.

8. Procédé selon l'une quelconque des revendications 1 à 7, dans lequel ladite au moins une polyamine est sélectionnée parmi le chitosane, l'éthylènediamine, la diéthylènetriamine, la triéthylènetétraamine, la tétraéthylènepentamine, la pentaéthylènehexamine, la dipropylènetriamine, la tripropylènetétramine, la tétrapropylènepentamine, la pentapropylènehexamine, et la polyéthylèneimine.

9. Procédé selon l'une quelconque des revendications 1 à 8, dans lequel ladite enzyme hydrolytique est la xynalase, l'amylase, la glucanase, la cellulase ou un mélange de celles-ci.

10. Procédé selon l'une quelconque des revendications 1 à 9, dans lequel à l'étape iii) un polyacrylamide, une carboxyméthylcellulose cationiques ou anioniques, ou un mélange de ceux-ci est en outre ajouté.

11. Procédé selon la revendication 9, dans lequel lesdits polyacrylamide, carboxyméthylcellulose cationiques ou anioniques ou un mélange de ceux-ci est présent en une concentration de 50 à 1 000 ppm dans le digesteur anaérobie.

12. Procédé selon l'une quelconque des revendications 10 ou 11, dans lequel lesdits polyacrylamide, carboxyméthylcellulose cationiques ou anioniques ou un mélange de ceux-ci et lesdites une ou plusieurs enzymes hydrolytiques supplémentaires se trouvent sous la forme d'un produit d'addition ou sont chimiquement liées les unes aux autres.

13. Utilisation d'une composition constituée d'au moins une enzyme hydrolytique et d'au moins une polyamine pour faire augmenter l'hydrolyse de polymères complexes contenus dans la biomasse dans le procédé selon l'une quelconque des revendications 1 à 12.

**14.** Utilisation selon la revendication 13, dans laquelle lesdites au moins une enzyme hydrolytique et au moins une polyamine sont sous la forme d'un produit d'addition ou sont chimiquement liées l'une à l'autre.

**15.** Utilisation de la composition selon l'une quelconque des revendications 13 ou 14, comprenant en outre un polyacrylamide, une carboxyméthylcellulose cationiques ou anioniques, ou un mélange de ceux-ci.

**16.** Utilisation de la composition selon l'une quelconque des revendications 13 à 15 ladite composition se trouvant sous la forme solide sélectionnée parmi une poudre, des billes, des flocons, des capsules, des bâtonnets, des barres, des pastilles, de la matière multiparticulaire, de la matière particulaire micronisée, des agglomérats, des microgranules, des granules ou des comprimés.

**17.** Utilisation de la composition selon l'une quelconque des revendications 13 à 15, ladite composition se trouvant sous la forme d'un liquide, d'un gel, d'une suspension ou d'une émulsion.

**EP 3 083 971 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KHEMKHAO et al.** *Bioresource Technology,* 2011, vol. 102, 4674-4681

- **ROSEN S.L.** Fundamental principles of polymeric materials. John Wiley and Sons, 1993, 58-68